# EUROPEAN PATENT APPLICATION

(11) **EP 3 031 458 A1**
(43) Date of publication of application: **15.06.2016**
(21) Application number: 14382502.4
(22) Date of filing: 09.12.2014
(51) Int. Cl.: A61K 31/427, A61P 25/18, A61P 25/28

(54) **Use of 1-[(2-phenylcycloprop-1-yl)carbonyl]-2-[(1,3-thiazolidin-3-yl)carbonyl]perhydroindole in the treatment of schizophrenia-associated cognitive deficits**

(71) Applicant: Iproteos S.L., 08028 Barcelona (ES)
(72) Inventor: Tarragó Clua, Teresa, 08028 Barcelona (ES); Prades Cosano, Roger, 08028 Barcelona (ES)
(74) Representative: ABG Patentes, S.L.

(57) **Abstract**

The present invention relates to the use of 1-[(2-phenylcycloprop-1-yl)carbonyl]-2-[(1,3-thiazolidin-3-yl)carbonyl]perhydroindole and pharmaceutical compositions thereof in the treatment of and/or prophylaxis of schizophrenia, particularly in the treatment of and/or prophylaxis of cognitive disorders associated to schizophrenia.

## Description

### FIELD OF THE INVENTION

The present invention relates to the use of 1-[(2-phenylcycloprop-1-yl)carbonyl]-2-[(1,3-thiazolidin-3-yl)carbonyl]perhydroindole or a pharmaceutically acceptable salt, stereoisomer or solvate thereof, in the treatment and/or prophylaxis of schizophrenia, particularly in the treatment and/or prophylaxis of cognitive disorders associated to schizophrenia. The invention also provides pharmaceutical compositions comprising 1-[(2-phenylcycloprop-1-yl)carbonyl]-2-[(1,3-thiazolidin-3-yl)carbonyl]perhydroindole.

### BACKGROUND

(2S,3aS,7aS)-1-[((1R,2R)-2-phenylcycloprop-1-yl)carbonyl]-2-[(1,3-thiazolidin-3-yl)carbonyl]perhydroindole, otherwise known as S17092, is a potent inhibitor of prolyl oligopeptidase (EC 3.4.21.26) (POP) (Barelli H et al., Biochem Biophys Res Commun. 1999;257(3):657-61).

POP is a serine protease that catalyses the hydrolysis of peptides at the C-terminal side of L-proline residues. It is widely distributed in mammals and can be purified from various organs, including the brain. The enzyme plays an important role in the breakdown of proline-containing neuropeptides related to learning and memory functions (Wilk S et al., Life Sci. 1983;33:2149-57; O'Leary RM, O'Connor B, J. Neurochem. 1995;65:953-63), and its activity has been reported to be altered (post-mortem) in several neurodegenerative diseases, including Alzheimer's disease (AD), Parkinson's disease, Huntington's disease and multiple sclerosis (MS) (Mantle D et al., Clin. Chim. Acta 1996;249(1-2):129-39).

In line with the above, the activity of S17092 in improving age-associated memory deficits and in inhibiting chemically-induced amnesia as well as cognitive deficits in chemically-induced Parkinson's disease has been demonstrated in various animal models (Morain P et al., CNS Drug. Rev. 2002;8(1):31-52; Schneider JS Neuropsychopharmacology 2002; 26(2):176-182).

The use of S17092 has also been connected to the treatment of other neural conditions such as bipolar disorder (WO 2006/102521 A2). However, the greater part of this literature remains speculative in nature and offers little or no realistic information pointing towards the feasibility of employing S17092 as a therapeutic agent.

Other publications predict the usefulness of S17092 in combination with a main therapeutic agent in the treatment of different neural disorders (WO 2010/098487 A1, US 2005/0171112 A1). However, yet again, no insight is provided as to the feasibility of employing S17092 as a medicament for patients suffering from such neural disorders.

An impending, real need exists to develop solid advances in the development of therapy for the treatment or prophylaxis of neural disorders or, equally importantly, of the symptoms associated to said neural disorders. This is especially imperative in those cases where current therapy is of limited value due to the lack of efficacy of treatments available, due to the appearance of serious side-effects after treatment and/or due to the inability of the treatments available to address the full range of symptoms associated with the disorder.

One of such disorders is schizophrenia. Unfortunately, diagnosis of schizophrenia is to date based solely on symptomatology as no reliable biomarker has yet been identified to detect the disorder. Schizophrenia is a mental disorder characterized by three main types of symptoms: positive symptoms, i.e. symptoms that non-schizophrenic individuals do not normally experience, such as delusions, disordered thoughts and speech, and tactile, auditory, visual, olfactory and gustatory hallucinations, typically regarded as manifestations of psychosis; negative symptoms, i.e. lacks of normal emotional responses or of other thought processes which non-schizophrenic individuals do show, such as flat expressions or little emotion, poverty of speech, inability to experience pleasure, lack of desire to form relationships, and lack of motivation; and cognitive dysfunction, such as deficits in attention and working memory that lead to an inability to organize one's life and to work effectively (Kellendonk C et al., Trends in Neurosciences 2009;32(6):347-358).

Particularly alarming is the fact that current first-line treatments for schizophrenia, which are almost always antipsychotic medication, whilst addressing positive and/or negative symptoms, generally fail to significantly improve the cognitive dysfunction associated to the disorder (National Institute for Health and Care Excellence, Clinical guideline 178 Psychosis and schizophrenia in adults: treatment and management, 2014**;** O'Carroll R, Advances in Psychiatric Treatment 2000; 6:161-168; Michalapoulou, European Neuropsychopharmacology 2013;.23:790-798 and Preskorn S.H., Journal of Psychiatric Practice 2014, 20(1): 12-24).

### BRIEF DESCRIPTION OF THE INVENTION

The present inventors have now surprisingly found that 1-[(2-phenylcycloprop-1-yl)carbonyl]-2-[(1,3-thiazolidin-3-yl)carbonyl]perhydroindole (structure represented by formula (I) below), or a pharmaceutically acceptable salt, stereoisomer or solvate thereof may be successfully used to address cognitive deficits associated with schizophrenia.

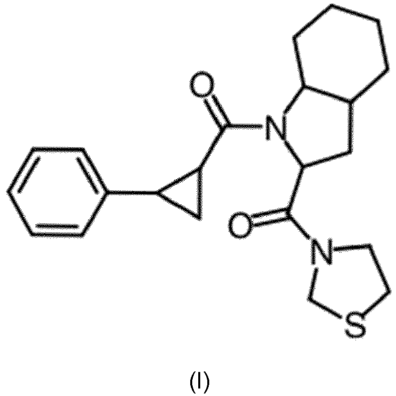

Therefore, one aspect of the invention relates to a compound of formula (I) or a pharmaceutically acceptable salt, stereoisomer or solvate thereof, for use in the treatment and/or prophylaxis of a cognitive disorder associated with schizophrenia.

Another aspect of this invention refers to a method for the treatment and/or prophylaxis of a cognitive disorder associated with schizophrenia in a mammal wherein a therapeutic amount of a compound of formula (I), or of a pharmaceutically acceptable salt, stereoisomer or solvate thereof, is administered to a patient in need of said treatment.

Another aspect of this invention refers to the use of a compound of formula (I), or of a pharmaceutically acceptable salt, stereoisomer or solvate thereof, for the preparation of a medicament, particularly for the prophylaxis and/or treatment of a cognitive disorder associated to schizophrenia.

Another aspect of this invention refers to a pharmaceutical composition comprising a compound of formula (I) or of a pharmaceutically acceptable salt, stereoisomer or solvate thereof; and a pharmaceutically acceptable carrier, adjuvant or vehicle; for use in the treatment and/or prophylaxis of a cognitive disorder associated with schizophrenia.

In a particular embodiment of each of the above aspects, the cognitive disorder associated with schizophrenia is at least one of working memory deficit, attention deficit, learning disorder, agnosia, anosognosia, amnesia, aphasia, apraxia, delirium and dementia.

Another aspect of the invention refers to a pharmaceutical composition comprising a compound of formula (I) or of a pharmaceutically acceptable salt, stereoisomer or solvate thereof; a pharmaceutically acceptable carrier, adjuvant or vehicle; and at least one other drug for the treatment of schizophrenia.

The above aspects and their preferred embodiments are additionally defined in the claims.

### DESCRIPTION OF THE FIGURES

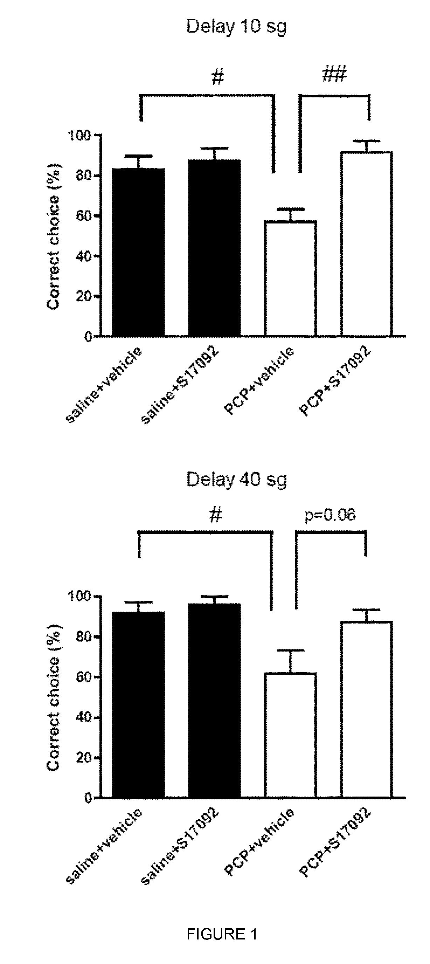

Figure 1 shows the effect of chronic treatment with Phencyclidine (PCP) on choice accuracy in the discrete-trial delayed spatial alternation task (T-maze). Animals were tested at two delay intervals: (top) 10 s and (bottom) 40 s. Mice treated with PCP (white bars) made significantly fewer correct choices than controls (black bars) (#P<0.05 Bonferroni's post hoc test of one-way ANOVA). S17092 reversed the PCP-impaired delayed alternation memory performance (percentage of correct trials) (#P<0.05, ##P<0.01 Bonferroni's post hoc test of one-way ANOVA). All data are presented as mean ± s.e.m. of 8 animals included in each experimental group.

### DETAILED DESCRIPTION OF THE INVENTION

In the context of the present invention, the following terms have the meaning detailed below.

As used herein the term "salt" must be understood as any form of a compound of formula (I) used in accordance with this invention in which said compound of formula (I) is in ionic form or is charged and coupled to a counter-ion.

The term "pharmaceutically acceptable salts" in the context of this invention means any salt that is tolerated physiologically (normally meaning that it is not toxic) when used in an appropriate manner for a treatment, applied or used, particularly, in humans and/or mammals.

In an embodiment, the pharmaceutically acceptable salt is formed by a cation (organic or inorganic) and an anionic form of a compound of formula (I). In a particular embodiment, the pharmaceutically acceptable salt is formed by a Li⁺, Na⁺, K⁺, Ca²⁺, Mg²⁺ or quaternary ammonium cation and an anionic form of a compound of formula (I).

In an embodiment, the pharmaceutically acceptable salt is formed by an anion (organic or inorganic) and a cationic form of a compound of formula (I). In a particular embodiment, the pharmaceutically acceptable salt is formed by a chloride, bromide, sulphate, methanesulfonate, formate, acetate, oxalate, succinate, malate, tartrate, mandelate, fumarate, lactate or citrate anion and a cationic form of a compound of formula (I).

The term "solvate" in accordance with this invention should be understood as meaning any form of a compound of formula (I) in which said compound of formula (I) is bonded by a non-covalent bond to another molecule (normally a polar solvent), including especially hydrates and alcoholates, like for example, methanolate. A preferred solvate is the hydrate.

The term "stereoisomer" in accordance with this invention may include enantiomers or diastereomers with respect to the chiral centers present in the compound of formula (I). The use of the single enantiomers or diastereoisomers and mixtures thereof fall within the scope of the present invention.

The compound of formula (I) may be prepared in racemic form, or individual enantiomers may be prepared either by enantiospecific synthesis or by resolution. These stereoisomers may be separated by conventional techniques known to the skilled person such as preparative chromatography.

Furthermore, a compound of formula (I) may exist in the form of its tautomers. Specifically, the term tautomer refers to one of two or more structural isomers of a compound of formula (I) that exist in equilibrium and are readily converted from one isomeric form to another, particularly amide-imidic tautomeric pairs.

The present invention also means to include within its scope the use of isotopically-labelled forms of a compound of formula (I), i.e. forms of a compound of formula (I) which differ only in the presence of one or more isotopically-enriched atoms, for example by replacement of at least one hydrogen by a deuterium or tritium atom, or the replacement of at least one carbon by ¹³C- or ¹⁴C-enriched carbon, or the replacement of at least one nitrogen by ¹⁵N-enriched nitrogen.

The compound of formula (I), or its salts, solvates or stereoisomers are preferably in pharmaceutically acceptable or substantially pure form. By pharmaceutically acceptable form it is meant, inter alia, that a compound of formula (I) has a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95%.

The preparation of salts, solvates and isomers can be carried out by methods known in the art.

In a particular embodiment of the different aspects of the present invention the compound of formula (I) or a pharmaceutically acceptable salt, stereoisomer or solvate thereof, is (2S,3aS,7aS)-1-[((1R,2R)-2-phenylcycloprop-1-yl)carbonyl]-2-[(1,3-thiazolidin-3-yl)carbonyl]perhydroindole or a pharmaceutically acceptable salt, stereoisomer or solvate thereof.

In a particular embodiment of the different aspects of the present invention a compound of formula (I) is used as such (i.e. not in the form of a pharmaceutically acceptable salt or solvate thereof). In a more specific embodiment, the compound of formula (I) used as such is (2S,3aS,7aS)-1-[((1R,2R)-2-phenylcycloprop-1-yl)carbonyl]-2-[(1,3-thiazolidin-3-yl)carbonyl]perhydroindole.

In a particular embodiment of the different aspects of the present invention a solvate (such as a hydrate) of a compound of formula (I) is used. In a more specific embodiment, the solvate of the compound of formula (I) is a solvate (such as hydrate) of (2S,3aS,7aS)-1-[((1R,2R)-2-phenylcycloprop-1-yl)carbonyl]-2-[(1,3-thiazolidin-3-yl)carbonyl]perhydroindole.

As used herein, the terms "treat", "treating" and "treatment" or the like include the eradication, removal, reversion, alleviation, modification, or control of a disorder.

As used herein, the terms "prophylaxis", "prevention", "preventing", "preventive", "prevent" or the like refer to avoiding, minimizing or hindering the onset or development of a disorder.

The term "cognitive disorder," as used herein, means any condition characterized by a deficit in mental activities associated with thinking, learning, or memory. In a particular embodiment of the invention, the cognitive disorder associated with schizophrenia to be treated and/or prevented is selected from working memory deficit, attention deficit, learning disorder, agnosia, anosognosia, amnesia, aphasia, apraxia, delirium and dementia. In a particular embodiment of the invention the cognitive disorder associated with schizophrenia is at least one of working memory deterioration, attention deficit and learning disorder, in particular working memory deterioration. In a particular embodiment of the invention the cognitive disorder associated with schizophrenia is working memory deficit.

The present invention further provides medicaments or pharmaceutical compositions comprising a compound of formula (I) or a pharmaceutically salt, stereoisomer or solvate thereof together with a pharmaceutically acceptable carrier, adjuvant, or vehicle, for administration to a patient.

As explained above, different kinds of symptoms underlie schizophrenia and current medication fails to significantly address all of said symptoms, particularly cognitive deficits. The present invention demonstrates the usefulness of compounds of formula (I) in addressing said cognitive deficits, whereas existing front-line medication primarily addresses the positive and/or negative symptoms of schizophrenia.

Therefore, the present invention additionally and advantageously refers to pharmaceutical compositions comprising a therapeutically effective amount of a compound of formula (I) or of a pharmaceutically acceptable salt, stereoisomer or solvate thereof; a pharmaceutically acceptable carrier, adjuvant or vehicle; and a therapeutically effective amount of at least one other drug for the treatment of schizophrenia. The present invention also refers to pharmaceutical compositions comprising a therapeutically effective amount of a compound of formula (I) or of a pharmaceutically acceptable salt, stereoisomer or solvate thereof; a pharmaceutically acceptable carrier, adjuvant or vehicle; and a therapeutically effective amount of at least one other drug for the treatment of schizophrenia; for use in the treatment and/or prophylaxis of schizophrenia.

In a particular embodiment, the at least one other drug is a typical antipsychotic.

In another particular embodiment, the at least one other drug is an atypical antipsychotic.

In another particular embodiment, the at least one other drug is selected from the group consisting of haloperidol, amisulpride, olanzapine, risperidone, clozapine, aripiprazole, iloperidone, ziprasidone, paliperidone, lurasidone, loxapine, thioridazine, asenapine, quetiapine, trifluoperazine, chlorpromazine, molindone and mesoridazine.

In a particular embodiment, the compound of formula (I) or a pharmaceutically acceptable salt, stereoisomer or solvate thereof, and the at least one other drug are contained in a single dosage form.

In a different embodiment, the compound of formula (I) or a pharmaceutically acceptable salt, stereoisomer or solvate thereof, and the at least one other drug are contained in separate dosage forms.

Particularly, the combination of a compound of formula (I) or of a pharmaceutically acceptable salt, stereoisomer or solvate thereof, and the at least one other drug may be formulated for its simultaneous, separate or sequential administration, with at least a pharmaceutically acceptable carrier, additive, adjuvant or vehicle. This has the implication that the combination of a compound of formula (I) or of a pharmaceutically acceptable salt, stereoisomer or solvate thereof and the at least one other drug may be administered:
a) As a combination that is being part of the same medicament formulation, both being then administered always simultaneously.
b) As a combination of two units, each with one of them giving rise to the possibility of simultaneous, sequential or separate administration. In a particular embodiment, a compound of formula (I) or a pharmaceutically acceptable salt, stereoisomer or solvate thereof is independently administered from the at least one other drug (i.e. at least in two units) but at the same time. In another particular embodiment, a compound of formula (I) or a pharmaceutically acceptable salt, stereoisomer or solvate thereof is administered first, and then the at least one other drug is separately or sequentially administered. In yet another particular embodiment, the at least one other drug is administered first, and then the compound of formula (I) or the pharmaceutically acceptable salt, stereoisomer or solvate thereof is administered, separately or sequentially, as defined.

The pharmaceutically acceptable carrier, adjuvant or vehicle of the pharmaceutical compositions according to the present invention can be selected among excipients, support materials, lubricants, fillers, solvents, diluents, colorants, flavour conditioners such as sugars, antioxidants, binders, adhesives, disintegrants, anti-adherents, glidants and/or agglutinants. In the case of suppositories, this may imply waxes or fatty acid esters or preservatives, emulsifiers and/or carriers for parenteral application. The selection of these pharmaceutically acceptable carriers, adjuvants or vehicles and the amounts to be used will depend on the form of application of the pharmaceutical composition.

The pharmaceutical compositions according to the present invention may be in any form suitable for administration to humans and/or animals, preferably humans including infants, children and adults and can be produced by standard procedures known to those skilled in the art. Therefore, the pharmaceutical compositions in accordance with the invention is suitable for topical or systemic administration, particularly for dermal, transdermal, subcutaneous, intramuscular, intra-articular, intraperitoneal, intravenous, intra-arterial, intravesical, intraosseous, intracavernosal, intranasal, pulmonary, buccal, sublingual, ocular, intravitreal, percutaneous, rectal, vaginal, oral, epidural, intrathecal, intraventricular, intracerebral, intracerebroventricular, intracisternal, intraspinal, perispinal, intracranial administration or for delivery via needles or catheters. Oral and subcutaneous administration are particularly preferred.

The pharmaceutical compositions of the invention may be in oral form, either solid or liquid. Suitable dose forms for oral administration may be tablets, pills, caplets, gel caps, chewing gums, capsules, granules, drops, syrups or solutions and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulfate.

The pharmaceutical compositions of the invention may be in the form of products for non-parenteral intranasal administration, preferably in the form of products for intranasal administration. Typically intranasal administration is carried out by using nasal sprays, squeeze bottles, and liquid droppers as delivery devices. To be used with these devices, the pharmaceutical compositions are advantageously a liquid solution or suspension.

The pharmaceutical compositions of the invention may be prepared by conventional methods of blending, filling or tabletting. Repeated blending operations may be used to distribute the active agent(s) throughout the composition employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositions may also be adapted for parenteral administration, e.g. as sterile solutions, suspensions or reconstitutable dry preparations, aerosols or sprays in the appropriate unit dosage form. Adequate excipients can be used, such as bulking agents, buffering agents or surfactants.

The pharmaceutical compositions of the invention may be formulated as a deposit in dissolved form or in patches, for percutaneous application.

Skin applications include ointments, gels, creams, lotions, suspensions or emulsions.

Suitable form of rectal application is by means of suppositories.

The mentioned pharmaceutical compositions will be prepared using standard methods such as those described or referred to in the European and US Pharmacopoeias and similar reference texts.

The physician or other person skilled in the art will determine the dosage of the compound of formula (I), or of the compound of formula (I) and the at least one other drug, which will be most suitable for the form of administration of the pharmaceutical composition chosen and for the patient under treatment. For instance, when the pharmaceutical composition is administered orally, larger quantities of the active agent will be required to produce the same effect as a smaller quantity given parenterally.

The following examples are merely illustrative of certain embodiments of the invention and cannot be considered as restricting it in any way.

### EXAMPLES

### S17092: (2S,3aS,7aS)-1-[((1R,2R)-2-phenylcycloprop-1-yl)carbonyl]-2-[(1,3-thiazolidin-3-yl)carbonyl]perhydroindole

S17092 is commercially available (e.g. Sigma-Aldrich, CAS Number 176797-26-5). S17092 may also be obtained by synthetic procedures described in the art (EP 0541407 B1 (Example 56); Portevin B et al., J. Med. Chem. 1996;39:2379-2391 (compound 54)).

### EFFECT OF S17092 ON LEARNING AND MEMORY IN A COGNITION IMPAIRMENT ANIMAL MODEL

S17092 was evaluated for its efficacy as cognition enhancer in a pharmacological model for cognitive impairment. The effects of S17092 were evaluated in untreated and PCP-treated mice. PCP is a non-competitive antagonist of the N-Methyl-D-aspartate (NMDA) receptor with an ability to produce a range of symptoms in rodents remarkably similar to those of patients suffering from schizophrenia (Jones CA et al., British Journal of Pharmacology 2011;164:1162-1194; Neill JC et al., Pharmacology & Therapeutics 2010;128:419-432; Morris BJ et al., Current Opinion in Pharmacology 2005;5:101-106; Kellendonk C et al., Trends in Neurosciences 2009;32(6):347-358).

In order to determine whether S17092 could act as cognitive enhancer, its ability to restore normal cognitive behavior was tested through the T-maze alternation task (Boess FG et al., Neuropharmacology 2004;47:1081-92; Spowart-Manning L et al., Behav. Brain Res. 2004;151:37-46). The protocol followed as well as the results observed will now be described in further detail.

### METHODOLOGICAL APPROACHES AND EXPERIMENTAL DESIGNS

### Animals

Experiments were conducted using C57/BL6 male mice aged 8-10 weeks. Mice were maintained at a constant temperature of 22°C with free access to food (commercial diet for rodents A04 Panlab, Barcelona, Spain) and water. Animals were housed in standard laboratory cages; each one containing groups of 8 individuals. All experiments procedures were carried out between 09:00 and 13:00 h. Behavioral tests were carried out under the same conditions. All animal studies were performed in accordance with the European Directive for the Protection of Vertebrate Animals used for experimental and Other Scientific Purposes (European Union Directive 2010/63/UE) and the Guide for the Care and Use of Laboratory Animals, 8^{th} edition published by the National Institutes of Health.

### T-maze alternation task test

Working memory was tested using a T-maze alternation task. The experiments were performed in a T-maze constructed of wood and painted brown. The walls were 15 cm high, and the alleys were 10.5 cm wide. The length of the main alley was 28 cm, and the length of the side alleys was 22.5 cm. The side alleys were closed off from the main alley by movable doors. A week before habituation, all animals were partially food restricted (each animal received 2 g of food per day) and remained that way throughout the remaining part of the experiment. This maintained each animal above 85% of its free-feeding body weight. The T-maze was cleaned between different animals but not between different trials. The food reward was a 14 mg cat pellet. The full experiment consisted of three parts: habituation, training, and testing. During habituation, all animals were placed on the T-maze until they ate two pieces of food or 90 s had elapsed. The food was spread randomly across the maze in order to encourage exploration This was repeated three times a day for 5 days. During training, all animals received six trials a day. Each trial consisted of two runs: a forced run and a free run. On the forced run, mice were forced to obtain a piece of food from one goal arm of the T-maze, with the other goal arm blocked by its door. Animals were then placed back into the start arm for a 10 s delay period. At the beginning of the free run, the mice were allowed to choose either goal arm. If the mice chose the arm opposite to the one they had been forced into during the forced run, they received the food reward. If the mice chose the same arm into which they had been forced, they received no food reward. There was a 5 min intertrial interval. The training period ended after control animals made 70% correct choices on 2 consecutive days. Mice were then tested for their performance at 10 or 40 s delay periods. Mice were given three 10 s delay and three 40 s delay trials during the day of testing, 30 min after drug exposure. The sequence of delays and forced-run food locations (left or right) were randomized each day, with the stipulation that the same delay or the same forced-arm location could not be used for three trials in a row. Mice were then tested for their performance in the maze recording the number of correct entries. Goal entries were defined as placing four paws in the arm.

### Drug administration

Phencyclidine (PCP, Sigma Aldrich) was administered at 10 mg/kg subcutaneously (s.c.) for 10 days. Control group was daily injected with saline s.c. Behavioral tests were carried out after a five-day washout period. On the test day, 30 min before the experiment, control and PCP-treated animals were acutely injected with a single dose of:
- S17092 at 5 mg/kg ip diluted in Tween80® 5% in saline; or
- Vehicle (5% Tween80® in saline) i.p.

### Data analyses

Data were analyzed with GraphPad PrismTM 5.0 (GraphPad Software, San Diego, CA, USA). Results are expressed as mean ± s.e.m. values. Results were submitted to a previous test for outliers detection (Grubbs' test). Data were analyzed using unpaired Student's t-test, one-way or two-way ANOVA tests followed by Bonferroni or Dunnett's post hoc test for multiple comparisons. Level of significance was set to P<0.05.

### EVALUATION OF PHARMACOLOGICAL ACTIVITY OF ACUTE S17092 ADMINISTRATION ON BEHAVIORAL DYSFUNCTIONS INDUCED BY SUBCHRONIC PHENCYCLIDINE (PCP) IN MOUSE

In order to evaluate working memory, animals treated chronically with PCP (10 mg/kg) and their controls were subjected to T-maze test. Animals were tested at two delay intervals. At 10 s and 40 s delay intervals, animals treated with PCP made significantly fewer correct choices (Figure 1). When administered to control animals, S17092 (5 mg/kg) induced a percentage of errors similar to those observed in the vehicle group (Figure 1). Acute injection of S17092 (5 mg/kg) reversed the deficits induced by PCP administration at 10 s and 40 s intervals (Figure 1). The results indicate a clear working memory dysfunction induced by PCP that is reversed by acute administration of S17092.

## Claims

1. A compound of formula (I) 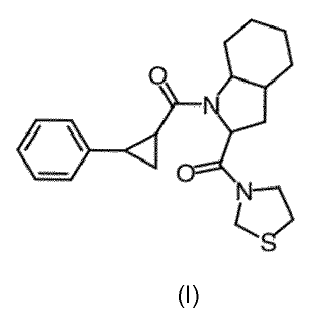 or a pharmaceutically acceptable salt, stereoisomer or solvate thereof, for use in the treatment and/or prophylaxis of a cognitive disorder associated with schizophrenia.

2. A compound of formula (I) or a pharmaceutically acceptable salt, stereoisomer or solvate thereof, for use according to claim 1, wherein the cognitive disorder associated with schizophrenia is at least one of working memory deficit, attention deficit, learning deficit, agnosia, anosognosia, amnesia, aphasia, apraxia, delirium and dementia.

3. A compound of formula (I) or a pharmaceutically acceptable salt, stereoisomer or solvate thereof, for use according to claim 1, wherein the cognitive disorder associated with schizophrenia is at least one of working memory deterioration, attention deficit and learning disorder.

4. A compound of formula (I) or a pharmaceutically acceptable salt, stereoisomer or solvate thereof, for use according to any one of the preceding claims wherein the compound of formula (I) is (2S,3aS,7aS)-1-[((1R,2R)-2-phenylcycloprop-1-yl)carbonyl]-2-[(1,3-thiazolidin-3-yl)carbonyl]perhydroindole.

5. A compound of formula (I) for use according to any of claims 1-3 wherein the compound is in free form (not in the form of a salt or a solvate).

6. A compound of formula (I) for use according to any of claims 1-3, wherein the compound of formula (I) is (2S,3aS,7aS)-1-[((1R,2R)-2-phenylcycloprop-1-yl)carbonyl]-2-[(1,3-thiazolidin-3-yl)carbonyl]perhydroindole in free form (not in the form of a salt or a solvate).

7. A pharmaceutical composition comprising a therapeutically effective amount of a compound of formula (I) or of a pharmaceutically acceptable salt, stereoisomer or solvate thereof, and a pharmaceutically acceptable carrier, adjuvant or vehicle, for use in the treatment and/or prophylaxis of a cognitive disorder associated with schizophrenia.

8. A pharmaceutical composition comprising:
• a compound of formula (I) or of a pharmaceutically acceptable salt, stereoisomer or solvate thereof;
• a pharmaceutically acceptable carrier, adjuvant or vehicle; and
• at least one other drug for the treatment of schizophrenia.

9. A pharmaceutical composition according to claim 8, wherein the at least one other drug for the treatment of schizophrenia is a typical antipsychotic or an atypical antipsychotic.

10. A pharmaceutical composition according to claim 8, wherein the at least one other drug for the treatment of schizophrenia is haloperidol, amisulpride, olanzapine, risperidone, clozapine, aripiprazole, iloperidone, ziprasidone, paliperidone, lurasidone, loxapine, thioridazine, asenapine, quetiapine, trifluoperazine, chlorpromazine, molindone or mesoridazine.

11. A pharmaceutical composition according to any one of claims 8 to 10 or a pharmaceutical composition for use according to claim 7, wherein the pharmaceutical composition is for dermal, transdermal, subcutaneous, intramuscular, intra-articular, intraperitoneal, intravenous, intra-arterial, intravesical, intraosseous, intracavernosal, intranasal, pulmonary, buccal, sublingual, ocular, intravitreal, percutaneous, rectal, vaginal, oral, epidural, intrathecal, intraventricular, intracerebral, intracerebroventricular, intracisternal, intraspinal, perispinal, intracranial administration or for delivery via needles or catheters.

12. A pharmaceutical composition according to any one of claims 8 to 11 or a pharmaceutical composition for use according to claim 7 or 11, wherein the pharmaceutical composition is for oral or subcutaneous administration.
